# EUROPÄISCHE PATENTSCHRIFT

(11) **EP 2 047 855 B1**
(45) Veröffentlichungstag und Bekanntmachung des Hinweises auf die Patenterteilung: **25.08.2010**
(21) Anmeldenummer: 07794063.3
(22) Anmeldetag: 21.06.2007
(51) Int. Cl.: A61K 33/38, A61K 31/195, A61K 31/455, A61K 31/132, A61K 33/04, A61K 31/282, A61P 35/00, A61P 31/04, A61P 31/12, A61K 9/08

(54) **ANTITUMORALE, ANTIBAKTERIELLE UND ANTIVIRALE PHARMAZEUTISCHE ZUSAMMENSETZUNG (VARIANTEN)**
ANTI-TUMORAL, ANTIBACTERIAL AND ANTIVIRAL PHARMACEUTICAL COMPOSITION (VARIANTS)
COMPOSITION ANTITUMORALE, ANTIBACTÉRIENNE ET ANTIVIRALE (ET VARIANTES)

(30) Priorität: 12.07.2006 RU 2006125085
(43) Veröffentlichungstag der Anmeldung: 15.04.2009
(73) Patentinhaber: Badulin, Nikolay Aleksandrovich, Moscow 127322 (RU)
(72) Erfinder: PLOTNIKOV, Vladimir Mikhaylovich, Tomsk, 634014 (RU)
(74) Vertreter: Jeck, Anton
(86) Internationale Anmeldenummer: PCT/RU2007/000341
(87) Internationale Veröffentlichungsnummer: WO 2008/007999

(56) Entgegenhaltungen:
- RU-C1- 2 094 048
- RU-C1- 2 114 858
- RU-C1- 2 242 977
- RU-C2- 2 181 050
- MASHKOVSKY M.D.: 'NOVAYA VOLNA' LEKARSTVENNYE SREDSTVA. POSOBIE DLYA VRACHEI. MOSCOW, OOO vol. 2, 2001, pages 87 - 89

## Beschreibung

Die Erfindung betrifft eine Zusammensetzung nach dem Oberbegriff des Anspruchs 1 oder 2.

Die vorgeschlagene Zusammensetzung und ihre Ausführungsformen sind in der Medizin und der Tierheilkunde, und zwar als Stoffe mit komplexer antiblastomatöser, antibakterieller und Antivirus-Wirkung einsetzbar. Diese Zusammensetzung kann in der Pharmazeutik bei der Herstellung von medizinischen und tierärztlichen Präparaten verwendet werden.

Es ist eine antiblastomatöse und antibakterielle Zusammensetzung bekannt, welche Silberverbindungen enthält, insbesondere Silbernitrat Hexamethylentetramin und Tetrajodid-Hexamethylentetramin (Patent KO 2181050, IPC 7 A61K33/18, A61 K33/38, 2002). Die bekannte Zusammensetzung ist jedoch ziemlich giftig und weist darüber hinaus ziemlich schlechte Wasserlöslichkeit auf. Deswegen kann diese Zusammensetzung nicht zur Herstellung von therapeutischen Präparaten für intravenöse Injektion benutzt werden.

Es sind auch antiblastomatöse Zusammensetzungen auf der Basis von Platin (Patent RU 2074861, 1997.03.10 IPC 6 C07P15/00) und ihren Verbindungen bekannt, z. B. von Derivaten von Platin Cis-Diamindiimidazolyl Dichlorid. Diese Zusammensetzungen weisen antiproliferative und zytostatische Wirkung auf (Pat. RU 2114858, IPC6 C07P15/00, A61 K31/295, 1998). Die bekannten Zusammensetzungen sind jedoch auch sehr giftig. Sie üben außerdem keine komplexe Einwirkung auf den Organismus infolge der ungenügend ausgeprägten antibakteriellen Eigenschaften aus.

In Bezug auf die Bestimmung, die Gesamtheit der wesentlichen Merkmale und den erreichbaren Effekt ist der nächste Stand der Technik gegenüber der angemeldeten Lösung das zytostatische und virentötende Präparat, welches folgende Komponenten enthält (Gew. %): Hexamethylentetramin, oder Imidazol, oder Phenazon - 0,007 bis 0,70; Höllenstein - 0,0085 bis 0,85; Natriumthiosulfat -0,0284 - 2,84; Wasser oder physiologische Kochsalzlösung - bis zu 100 % (Patent RU 2094048, 1997).

Da in dem genannten Mittel Silbersalze als zytostatische Mittel benutzt werden, ist es ein moderneres fortschrittlicheres antiblastomatöses Mittel. Der wesentliche Mangel der Prototyp-Zusammensetzung ist jedoch ihre ungenügend hohe antiblastomatöse Wirkung, die durch die Toxizität des Höllensteins begrenzt ist. Der Höllenstein beeinträchtigt wesentlich die Lebewesen. Außerdem löst diese Zusammensetzung nicht die Aufgabe einer Komplextherapie, welche eine antibakterielle Einwirkung auf den Körper einschließt. Zugleich werden die malignen Tumore und Leukosen öfters (meistens) durch die bakteriellen Infektionen erschwert. Das lässt sich durch den Rückgang der Schutzfunktionen des Immunsystems des kranken Organismus erklären. Darunter fallen auch die toxische Nebenwirkung der bekannten Chemotherapeutika. Außerdem ist das Phenazon momentan aus der Produktion genommen, da es jetzt wirksamere Komponenten gibt.

Es ist Aufgabe der Erfindung, pharmazeutische Zusammensetzungen zu entwickeln, die eine starke ausgeprägte antiblastomatöse Wirkung und gleichzeitig eine antibakterielle und Antivirus-Wirkung auf den Körper aufweisen. Außerdem soll die zusätzliche toxische Wirkung der Arzneimittel auf den Organismus vermindert werden.

Die gestellte Aufgabe wird durch die Merkmale des Anspruchs 1 oder 2 gelöst.

Die Basis der pharmazeutischen Zusammensetzung besteht aus Silbersalzen, Hexamethylentetramin, Natriumthiosulfat und Wasser. Zusätzlich enthält die pharmazeutische Zusammensetzung die Alpha-Asparaginsäure oder Asparagin und Nikotinsäure bei folgendem Komponentenverhältnis in Gew. %:

| | |
|---|---|
| Höllenstein | 0,016-1,6, |
| Hexamethylentetramin | 0,03 - 1,4, |
| Natriumthiosulfat | 0,02 - 5,0, |
| Alpha-Asparaginsäure oder Asparagin | 0,07 - 0,70, |
| Nikotinsäure | 0,07 - 0,70 und |
| Wasser | Rest bis 100. |

Eine andere Ausführungsvariante ist eine zusätzliche Einführung in die Zusammensetzung eines imidazolhaltigen Platins von Cis-[Pt(NH₃)₂Im₂]Cl₂ oder Cis-[Pt(NH₂OH)₂Im₂]Cl₂ mit folgendem Mengengehalt in Gew. %:

| | |
|---|---|
| Höllenstein | 0,016-1,6, |
| Hexamethylentetramin oder Imidazol | 0,03 -1,4, |
| Cis-[Pt(NH₃)₂Im₂]Cl₂ oder Cis-[Pt(NH₂OH)₂Im₂]Cl₂ | 0,056 - 5,6, |
| Alpha-Asparaginsäure oder Asparagin | 0,07 - 0,70, |
| Nikotinsäure | 0,07 - 0,70 und |
| Wasser | Rest bis 100. |

Die Ausführung der neuen Zusammensetzung auf der Basis von Platinverbindungen verteuert zwar den Preis des Präparats. Dabei wird jedoch die Erhöhung der antiblastomatösen Wirkung des Präparats unter gleichzeitiger Verminderung der Giftigkeit erreicht. Das macht diese Zusammensetzung vorteilhafter in Bezug auf ihre medizinische Anwendung.

In der angemeldeten Gruppe der Zusammensetzungen verursacht die Vergrößerung des Anteils der antiblastomatösen und bakteriziden Hauptkomponente, nämlich des Höllensteins innerhalb von 0,016-1,6 keine Erhöhung der allgemeinen toxischen Einwirkung der Zusammensetzung. Dies wird dank der Einführung von Hexamethylentetramin, Alpha-Asparaginsäure oder Asparagin und Nikotinsäure innerhalb der angemeldeten Anteile erreicht. Die aufgezählten Komponenten wirken auf die Lebewesen als synergetische Verbindungen ein, die die toxische Wirkung des Höllensteins mindern. Diese Wirkungsweise wird bei der ersten Ausführungsform der Zusammensetzung durch Natriumthiosulfat und bei der zweiten Ausführungsform durch Hexamethylentetramin oder Imidazol sichergestellt.

Das Vorhandensein der Platinverbindungen bei der zweiten Ausführungsform der Zusammensetzung erhöht ebenfalls die antiblastomatöse Wirkung unter gleichzeitiger Verminderung der Giftigkeit. Das bevorzugt die Zusammensetzung bei ihrer medizinischen Anwendung mehr. Die erste Ausführungsvariante der Zusammensetzung ist bei ihrer Anwendung in der Veterinärmedizin mehr gefragt, da diese Zusammensetzung billiger ist.

Die Analyse der Arzneimittel ist vorgenommen und festgestellt worden, dass in den zugänglichen Patent- und medizinischen Nachschlagquellen keine Vergleichserzeugnisse erwähnt sind, welche durch ihre Gesamtheit der Merkmale gekennzeichnet sind, die allen kennzeichnenden Merkmalen der angemeldeten Substanz identisch sind. Folglich sind die Zusammensetzungen neu.

Es wurde eine Recherche nach bekannten Zusammensetzungen in diesem und in den angrenzenden technischen Bereichen durchgeführt, um die gleichen Merkmale zu finden, die mit denen des Prototyps zusammenfallen. Während der Recherche wurden keine Zusammensetzungen festgestellt, deren Merkmale mit den kennzeichnenden Merkmalen der Erfindung völlig zusammenfallen. Die Bekanntheit und die Offenbarkeit des Einflusses der angemeldeten kennzeichnenden Merkmale auf die Erreichung des genannten technischen Ergebnisses wurde nicht nachgewiesen.

Die Erfindung wird anhand von in den Zeichnungen dargestellten Diagrammen näher erläutert. Es zeigen:
- Fig. 1: Diagramme, die die Abhängigkeit der proliferativen Aktivität der bös- artigen Zellen von der angemeldeten Zusammensetzung innerhalb der minimalen Mengenanteile der Komponenten im Vergleich zum Prototyp darstellen,
- Fig. 2: Diagramme, die die Abhängigkeit der proliferativen Aktivität der bös- artigen Zellen von der angemeldeten Zusammensetzung innerhalb der optimalen Mengenanteile der Komponenten im Vergleich zum Prototyp darstellen und
- Fig. 3: Diagramme, die die Abhängigkeit der proliferativen Aktivität der bös- artigen Zellen von der angemeldeten Zusammensetzung innerhalb der maximalen Mengenanteile der Komponenten im Vergleich zum Prototyp darstellen.

Bei der Ausführung der Beispiele wurde eine Wasserlösung der Zusammensetzung verwendet. Diese Wasserlösung enthielt sowohl die bekannten als auch die neuen Komponenten bei ihren unterschiedlichen Kombinationen.

Tabelle 1 enthält Angaben über den Einfluss der angemeldeten Zusammensetzung auf die Zerstörung der bösartigen Zellen. Diese Angaben beweisen die antiblastomatöse Aktivität. Tabelle 2 enthält Angaben über die antiblastomatöse Aktivität der angemeldeten Zusammensetzung auf Lebewesen. Tabelle 3 enthält Angaben über die antibakterielle Aktivität der angemeldeten Zusammensetzung. In Tabelle 4 sind Angaben über die toxikologischen Untersuchungen nach der ersten und der zweiten Ausführungsvariante der angemeldeten Zusammensetzungen im Vergleich zum bekannten onkologischen Mittel auf Platin-Basis - Cis-Platin - angeführt. Tabelle 5 enthält die Vergleichsangaben der virentötenden Aktivität der Zusammensetzung nach der zweiten Ausführungsvariante. Tabelle 6 enthält Angaben über die Einschätzung der antiblastomatösen Aktivität der angemeldeten Zusammensetzung nach der zweiten Ausführungsvariante.

### Beispiel 1

0,016 g Höllenstein werden mit 0,03 g Hexamethylentetramin und 0,02 g Natriumthiosulfat vermengt. Dazu werden 0,07 g Alpha-Asparaginsäure und 0,07 g Nikotinsäure zugegeben, nachdem sie in der erforderlichen Wassermenge aufgelöst worden sind. Es wird auf die Trübheit der resultierenden Lösung hingewiesen. Die Zusammensetzung wird in Bezug auf die antiblastomatöse, antibakterielle und virentötende Aktivität untersucht.

### Beispiel 2

1 g Höllenstein, 0,8 g Hexamethylentetramin und 3 g Natriumthiosulfat werden miteinander gemischt. Danach werden 0,4 g Nikotinsäure und 0,4 g Alpha-Asparaginsäure oder Asparagin nach ihrer Auflösung in der erforderlichen Wassermenge dazugegeben. Die resultierende Lösung wird in Bezug auf ihre antiblastomatöse, antibakterielle und Antivirusaktivität untersucht.

### Beispiel 3

1,6 g Höllenstein, 1,4 g Hexamethylentetramin und 5 g Natriumthiosulfat werden miteinander vermengt und mit 0,7 g Asparagin und 0,7 g Nikotinsäure ergänzt. Dabei werden Asparagin und Nikotinsäure in der erforderlichen Wassermenge aufgelöst. Ein Teil der Reagenzien fällt aus. Die Zusammensetzung wird in Bezug auf ihre antiblastomatöse, antibakterielle und Antivirusaktivität untersucht.

Es wurde der Vergleich der zytostatischen antiblastomatösen Aktivität der angemeldeten Zusammensetzung nach der zweiten Ausführungsvariante (die Zusammensetzung mit den imidazolhaltigen Platinverbindungen) in Bezug auf die Geschwulstzellen untersucht. Dabei wurde ³H-Thymidin nach 24 Stunden der Inkubation (Impulszahl pro Sekunde) eingeführt. Die Radioaktivitätsmessung wurde anhand des Beta-Zählers Mark - III durchgeführt. Die Beurteilung der zytostatischen Aktivität der angemeldeten Zusammensetzung wurde an der Zellenkultur von menschlichen Erythroleukämie der Linie K-562 und NTZ (HLIT)-102 vorgenommen. Diese Zellen wurden im Nährboden RPMI-1640 (Serva) mit dem 10%-igen Kalbsembryoserum (2 x 10⁵ Zellen/ml) und in der kurzfristigen Zellenkultur der Rauscher-Leukämie und des Ehrlich-Karzinoms kultiviert. Die Untersuchung erfolgte in Bezug auf die Einführung von ³H-Thymidin in den Geschwulstzellen und auf die Einfärbung durch Trypanblau jeweils 24 Stunden und 48 Stunden nach der Inkubation.

### Beispiel 4

0,016 g Höllenstein werden mit 0,03 g Hexamethylentetramin vermengt und mit 0,056 g Cis-[Pt(NH₃)₂Im₂]Cl₂ ergänzt. Danach werden die in der erforderlichen Wassermenge aufgelösten 0,07 g Alpha-Asparaginsäure oder Asparagin und 0,07 g Nikotinsäure nachgesetzt. Die Zusammensetzung wird in Bezug auf die antiblastomatöse, antibakterielle und virentötende Aktivität untersucht.

### Beispiel 5

0,016 g Höllenstein werden mit 0,03 g Imidazol vermengt und mit 0,056 g Cis-[Pt(NH₃)₂Im₂]Cl₂, 0,07 g, der Alpha-Asparaginsäure oder Asparagin und 0,07 g der Nikotinsäure (minimaler Komponentengehalt), aufgelöst in der erforderlichen Wassermenge, ergänzt. Die Zusammensetzung wird in Bezug auf die antiblastomatöse, antibakterielle und virentötende Aktivität untersucht.

### Beispiel 6

0,016 g Höllenstein und 0,07 g Hexamethylentetramin werden miteinander vermischt und mit den in erforderlicher Wassermenge aufgelösten 0,0056 g Cis-[Pt(NH₂OH)₂Im₂]Cl₂, 0,07 g Alpha-Asparaginsäure oder Asparagin und 0,07 g Nikotinsäure ergänzt. Die Zusammensetzung wird in Bezug auf ihre antiblastomatöse, antibakterielle und virentötende Aktivität untersucht.

### Beispiel 7

1,6 g Höllenstein werden mit 0,7 g Imidazol vermengt. Dazu werden 5,6 g Cis-[Pt(NH₂OH)₂Im₂]Cl₂ oder Cis-[Pt(NH₃)₂Im₂]Cl₂, 0,07 g Alpha-Asparaginsäure oder Asparagin und 0,07 g Nikotinsäure (maximaler Komponentengehalt) nachgesetzt, die in der erforderlichen Menge von Wasser aufgelöst wurden. Es wurde Ausfällung und Instabilität der Lösung festgestellt. Die Zusammensetzung wird in Bezug auf antiblastomatöse, antibakterielle und virentötende Aktivität untersucht.

### Beispiel 8

1 g Höllenstein, 0,8 g Hexamethylentetramin und 4 g Cis-[Pt(NH₂OH)₂Im₂]Cl₂ oder Cis-[Pt(NH₃)₂Im₂]Cl₂ werden miteinander vermengt und mit 0,4 g Nikotinsäure und 0,4 g Alpha-Asparaginsäure oder Asparagin (optimaler Komponentengehalt), aufgelöst in der erforderlichen Wassermenge, ergänzt. Wenn das Komponentenverhältnis optimal ist und innerhalb des angemeldeten Bereichs liegt, wird eine stabile transparente Lösung erzeugt. Diese Lösung wird in Bezug auf antiblastomatöse und antibakterielle Aktivität untersucht.

### Beispiel 9

In der Zusammensetzung nach dem Prototyp werden 0,0085 g Höllenstein mit 0,007 g Hexamethylentetramin (Methenamin) vermengt und mit 0,0284 g Natriumthiosulfat-Hydrat ergänzt. Es wird eine durchsichtige Lösung gewonnen, welche in Bezug auf zytostatische und virentötende Aktivität untersucht wird.

### Beispiel 10

In der Zusammensetzung nach dem Prototyp werden 0,085 g Höllenstein mit 0,07 g Hexamethylentetramin vermengt und mit Wasserlösung von 0,284 g Natriumthiosulfat Hydrat ergänzt. Es wird eine durchsichtige Lösung gewonnen, welche in Bezug auf zytostatische und virentötende Aktivität untersucht wird.

### Beispiel 11

In der Zusammensetzung nach dem Prototyp werden 0,85 g Höllenstein mit 0,7 g Hexamethylentetramin vermengt und mit Wasserlösung von 2,84 g Natriumthiosulfat Hydrat ergänzt. Es wird eine durchsichtige Lösung gewonnen, welche in Bezug auf zytostatische und virentötende Aktivität untersucht wird.

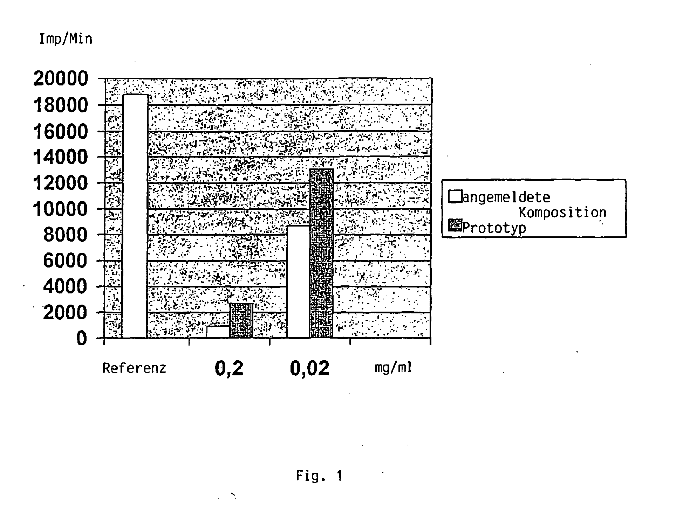

Die Materialien der Versuche zum Nachweis der Wirksamkeit der angemeldeten Merkmale zur Lösung der gestellten technischen Aufgabe sind in den Tabellen sowie in den Diagrammen in Fig.1, Fig.2 und Fig. 3 aufgeführt.

Die Tabelle 1 enthält die Ergebnisse der Versuche zur Ermittlung des Einflusses der angemeldeten Zusammensetzung auf die Geschwulstzellen. Daraus ist ersichtlich, dass die am besten ausgeprägte antiblastomatöse Wirkung die Zusammensetzung nach Beispiel 2 aufweist (praktisch vollständige Unterdrückung der bösartigen Zellen). Die Vergleichsdaten aus der Tabelle 1 zeugen davon, dass an der unteren Grenze des quantitativen Komponentenverhältnisses die Zusammensetzung nach dem Prototyp eine sehr geringe Wirkung auf die Testzellen K-562 und HUT-102 ausübt.

Ein mehr detaillierter Vergleich der angemeldeten Zusammensetzung mit dem Prototyp, welcher die unterschiedliche antiblastomatöse Wirkung bei verschiedenem quantitativen Komponentenverhältnis berücksichtigt, bestätigt das Erreichen des technischen Ergebnisses bei der Entwicklung der angemeldeten Zusammensetzung innerhalb des gesamten angemeldeten Bereichs des quantitativen Komponentenbereichs.

In Tabelle 2 sind Angaben über das Studium der antiblastomatösen Wirkung der angemeldeten Zusammensetzung an Mäusen CBA mit Ehrlich-Karzinom (bei dreimaliger intraabdominaler Injektion in der Dosis von 30 mg/kg) angeführt. Die Behandlung wurde 24 Stunden nach Umimpfung von 7x10⁶ der Tumorzellen je Maus begonnen. Der Einsatz der vorgeschlagenen Zusammensetzung trägt einer Verlängerung der Mauslebensdauer um das zweifache im Vergleich zu der Referenzgruppe (bei keiner Behandlung) bei.

Die Tabelle 2 kennzeichnet die antiblastomatöse Aktivität der angemeldeten Zusammensetzung in vivo für die angemeldete Zusammensetzung nach der ersten Ausführungsvariante. Wie aus den Angaben der Tabelle 2 ersichtlich ist, betrug die Lebensdauer der Mäuse mit Ehrlich-Karzinom in der Referenzgruppe 19,7 Tage. Bei der Behandlung unter Einsatz der angemeldeten Zusammensetzung nach der ersten Ausführungsvariante gemäß Beispiel 2 betrug die Lebensdauer 53,7 Tage. Dabei ist bekannt (Patent RU Nr. 2094048), dass der Einsatz der Prototyp-Zusammensetzung die Lebensdauer der Versuchstiere von 18,6 Tagen (Referenz) bis 41,3 Tage (behandelte Tiere) verlängert. Folglich verlängert die Prototyp-Zusammensetzung die Lebensdauer der Versuchstiere um das 2,22-fache. Der Einsatz der angemeldeten Zusammensetzung bei den unter ähnlichen Bedingungen durchgeführten Versuchen vergrößert die Lebensdauer der Versuchstiere um das 2,73-fache.

Somit trägt die Anwendung der vorgeschlagenen Zusammensetzung der Verlängerung der Lebensdauer der Mäuse um mehr als das 2-fache im Vergleich zu Referenztieren (bei keiner Behandlung) bei. Gleichzeitig wird die antiblastomatöse Aktivität im Vergleich zum Prototyp wesentlich erhöht.

Die antibakterielle Aktivität der angemeldeten Zusammensetzung wurde nach dem Verfahren einer seriellen Verdünnung der Präparate in den Bakterienkulturen unter Konzentration von 1 mg/ml und 10 mg/ml festgestellt. Die Präparate Cis-[Pt(NH₃)₂Im₂]Cl₂ oder Cis-[Pt(NH₂OH)₂Im₂]Cl₂ weisen ihrer Wirkungstendenz nach eine ähnliche Wirkung innerhalb der gestellten technischen Aufgabe auf. Zum Nachweisen der Antiviruswirkung der angemeldeten Zusammensetzung wurde sowohl Cis-[Pt(NH₃)₂Im₂]Cl₂ als auch [Pt(NH₂OH)₂Im₂]Cl₂ eingesetzt. Die Versuchsergebnisse 24 Stunden nach dem Wachstum der Mikroorganismen bei 37 °C sind in Tabelle 3 angeführt. Aus den Angaben der Tabelle folgt, dass die angemeldete Zusammensetzung eine ausgeprägte zytotoxische Einwirkung auf die Bakterienzellen aufweist. Dadurch wird eine 100%-ige Zerstörung der Bakterien bei den erforschten Konzentrationen verursacht (an der Bereichsgrenze bei optimaler Zusammensetzung 90 %, bei anderen Zusammensetzungen 50 bis 70 %).

Die Behandlung der Virus Rauscher Leukämie mit der Zusammensetzung in der Konzentration von 1 mg/ml und 10 mg/ml innerhalb von 4 Stunden mit einer nachfolgenden intraabdominalen Injektion des Virus-Inokulum bei BALB/c Mäusen hatte einen 100%-ige Schutzeffekt gegen Letalwirkung des Virus, ohne den Tod der Tiere zu verursachen.

Die Forschungen der akuten Toxizität der optimalen Zusammensetzung (Beispiele 2 und 8) wurden an den Mäusen der BALB/c-Linie bei intraabdominalen Injektion der Wasserlösung im Bereich von 15 mg/kg bis 120 mg/kg (Tabelle 4) vorgenommen. Die Tiere wurden innerhalb von 30 Tagen beobachtet. Zum Vergleich wurde das klinische Präparat Cis-Platin gewählt. Die Toxizität der angemeldeten Zusammensetzung war wesentlich geringer, als die des bekannten Präparats.

Die Anwendung der angemeldeten Zusammensetzung verhinderte die Entwicklung von Virus-Hämoblastose bei Versuchstieren: Der Anteil der Todesfälle bei den Versuchstieren betrug nur 8,5 Prozent (3 von 35 Tieren). Im Gegenteil zur Referenzgruppe von nicht behandelten Tieren (35 Mäuse), wurde ein 100%-iger Letaleffekt festgestellt.

Die Tabelle 5 enthält Vergleichsangaben der virentötenden Aktivität der angemeldeten Zusammensetzung nach der zweiten Ausführungsvariante am Beispiel der Inhibierung der Virus-Hämoblastose für die angemeldete Zusammensetzung mit minimalem (Beispiel 5), optimalem (Beispiel 8) und maximalem (Beispiel 7) quantitativen Komponentengehalt innerhalb der angemeldeten Bereiche.

Aus den Daten der Tabelle 5 folgt, dass der Anteil der verstorbenen Tiere beim optimalen quantitativen Komponentenbestand in der angemeldeten Zusammensetzung minimal ist. Dabei weist die Zusammensetzung ihre virentötende Aktivität innerhalb des gesamten angemeldeten Bereichs auf.

Die Tabelle 6 enthält Angaben über das Studium der antiblastomatösen Wirkung der angemeldeten Zusammensetzung an CBA-Mäusen mit Mastozytom P-815 (bei dreimaliger intraabdominaler Injektion der Zusammensetzung in der Dosis von 30 mg/kg). Aus der Tabelle ist ersichtlich, dass der Einsatz der vorgeschlagenen Zusammensetzung eine dreifache Verlängerung der Lebensdauer der Versuchstiere im Vergleich zur Referenzgruppe (nicht behandelter Tiere) verursacht.

Die antiblastomatöse zytostatische Wirkung der platinhaltigen Zusammensetzung nach den Beispielen 5, 8 und 7 für den minimalen, optimalen und maximalen quantitativen Komponentengehalt wird jeweils in den Fig. 1, 2 und 3 dargestellt. Es wurden die Konzentrationen von 0,02 und 0,2 mg/ml für die angemeldete Zusammensetzung benutzt. Die Einwirkungsergebnisse wurden mit denen des Prototyps unter gleichen Konzentrationen verglichen. In den Diagrammen ist die Wirkung der angemeldeten Zusammensetzung auf die mit Rauscher-Leukämie betroffenen Zellen in Bezug auf die Einführung von ³H-Thymidin 24 Stunden nach der Inkubation im Vergleich mit der Referenzgruppe abgebildet (Imp/Min). Die Referenzgruppe, d. h. die Züchtung der bösartigen Zellen unter den gleichen Bedingungen ohne Einwirkung der platinhaltigen Zusammensetzung, beträgt 18750 Imp/Min. Aus den Diagrammen ist ersichtlich, dass die angemeldete Zusammensetzung (helle Diagrammbalken) innerhalb des gesamten angemeldeten Bereichs einen ausgeprägteren Inhibierungseffekt gegenüber der Prototyp-Zusammensetzung (dunklere Balken) aufweist. Das ermöglicht, die Proliferation der bösartigen Zellen fast völlig zu unterdrücken. Für alle zitierten Zusammensetzungen aus den Beispielen 5, 7 und 8 wurde ein dosisabhängiger Effekt der Unterdrückung der Proliferation von bösartigen Zellen festgestellt.

Aus dem Diagramm in Fig. 1 folgt, dass die zytostatische Wirkung (in Imp/Min) beim Einsatz der angemeldeten Zusammensetzung (mit dem Komponentengehalt innerhalb des im Beispiel 5 genannten Bereichs) und der Zusammensetzung nach dem Prototyp (mit dem Komponentengehalt gemäß dem Beispiel 9) sich folgenderweise ausprägt: Bei der Konzentration von 0,2 mg/ml 930 für die angemeldete Zusammensetzung und 2700 für den Prototyp; bei der Konzentration von 0,02 mg/ml 8700 für die angemeldete Zusammensetzung und 13100 für den Prototyp.

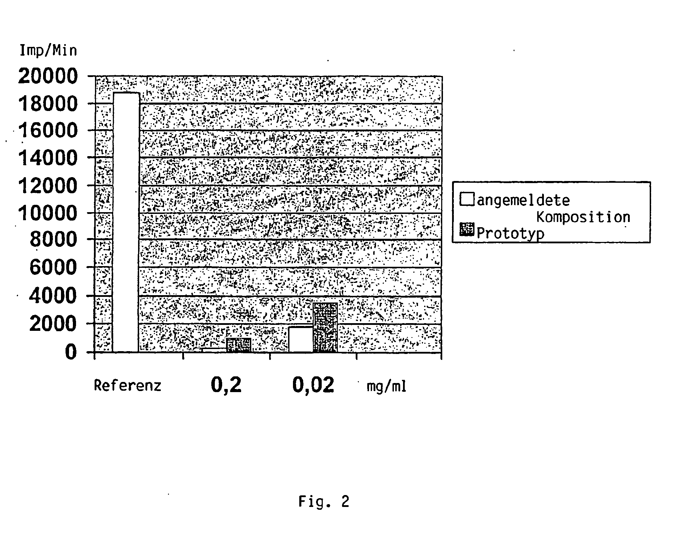

Die Diagramme aus Fig. 2 enthalten die Vergleichsgrößen der zytostatischen Aktivität der angemeldeten Zusammensetzung (Beispiel 8) mit optimalem Komponentengehalt und der Zusammensetzung nach dem Prototyp innerhalb der Komponenten (Beispiel 10). Daraus ist ersichtlich, dass die zytostatische Wirkung (Imp/Min) bei der Konzentration von 0,2 mg/ml für die angemeldete Zusammensetzung 310 und für den Prototyp 1000 beträgt. Bei der Konzentration von 0,02 mg/ml beträgt die zytostatische Wirkung (Imp/Min) für die angemeldete Zusammensetzung 1770 und für den Prototyp 3480.

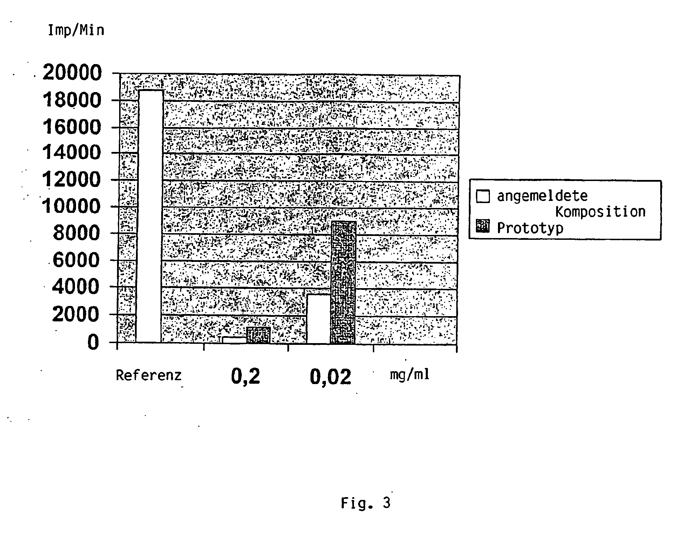

Die Diagramme in Fig. 3 enthalten die Vergleichsgrößen der zytostatischen Aktivität der angemeldeten Zusammensetzung (Beispiel 7) mit maximalem Komponentengehalt und der Zusammensetzung nach dem Prototyp (Beispiel 11) bei einer Konzentration von 0,2 mg/ml. Daraus ist ersichtlich, dass die zytostatische Wirkung (Imp/Min) bei der Konzentration von 0,2 mg/ml für die angemeldete Zusammensetzung 460 (Imp/Min) und für den Prototyp 1150 und bei der Konzentration von 0,02 mg/ml für die angemeldete Zusammensetzung 3600 und für den Prototyp 9000 beträgt.

Außerdem wurde eine spezielle Serie von Versuchen im Zusammenhang mit dreimaliger Injektion der platinhaltigen Zusammensetzung (intraabdominale Injektion in der Dosis von 30 mg/kg) bei 35 Mäusen mit Rauscher-Leukämie 24 Stunden nach der Umimpfung des Virus-Inokulums vorgenommen. Die Anwendung der angemeldeten platinhaltigen Zusammensetzung verhinderte eine verzögerte Entwicklung von Hämoblastose bei den Versuchstieren. Der Überlebensanteil betrug 90 Prozent im Gegenteil zu der Referenzgruppe der unbehandelten Tiere (35 Mäuse), bei denen ein 100%-iger Letaleffekt festgestellt wurde.

Für die angemeldete Zusammensetzung und ihre Ausführungsformen ist ihre hohe antiblastomatöse Effizienz sowie die Fähigkeit, systematisch und umfassend auf die Versuchstiere und -Zellenkulturen einzuwirken, bewiesen. Dabei ist die toxische Einwirkung der pharmazeutischen Zusammensetzung und ihrer Ausführungsvarianten weniger ausgeprägt im Vergleich zu den bekannten Behandlungsmitteln. Die angemeldete Zusammensetzung und ihre Ausführungsformen können in der Pharmazie bei der Herstellung von medizinischen und tierärztlichen Präparaten eingesetzt werden.

### Antiblastomatöse zytotoxische Aktivität der angemeldeten Zusammensetzung in vitro

**Tabelle 1**

| Gestorbene Zellen, % | | | | | | | | | | | | | |
|---|---|---|---|---|---|---|---|---|---|---|---|---|---|
| Art der Zellen | Angemeldete Zusammensetzung, mg/ml | | | | | | Prototyp, mg/ml | | | | | | Referenz |
| | nach Beispiel 1 | | nach Beispiel 2 | | nach Beispiel 3 | | nach Beispiel 9 | | nach Beispiel 10 | | nach Beispiel 11 | | |
| | 1 | 0,1 | 1 | 0,1 | 1 | 0,1 | 1 | 0,1 | 1 | 0,1 | 1 | 0,1 | - |
| K-562 | 65 | 30 | 100 | 100 | 100 | 70 | 29 | 15 | 90 | 65 | 85 | 60 | 8 |
| HUT-102 | 50 | 25 | 100 | 95 | 100 | 60 | 24 | 14 | 80 | 55 | 70 | 50 | 7 |

### Lebensdauer der Mäuse mit Ehrlich-Karzinom (in Tagen)

**Tabelle 2**

| Angemeldete Zusammensetzung (Ausführungsvariante 1, Beispiel 2) | Prototyp | Referenz (unbehandelte Mäuse) |
|---|---|---|
| 53,7 ± 4,9 | 44,2 ± 3,1 | 19,7 ± 1,8 |

### Antibakterielle Aktivität der angemeldeten Zusammensetzung

**Tabelle 3**

| Angemeldete Zusammensetzung | mg/ml | % der Unterdrückung des Bakterienwachstums | | | |
|---|---|---|---|---|---|
| | | Staphylokokkus | Streptokokkus | Kolibakterium | Tuberkulose mykobakterien |
| nach Beispiel 1 | 1 | 60 | 60 | 70 | 50 |
| | 10 | 100 | 100 | 100 | 100 |
| nach Beispiel 2 | 1 | 100 | 100 | 100 | 100 |
| | 10 | 100 | 100 | 100 | 100 |
| nach Beispiel 3 | 1 | 90 | 100 | 100 | 70 |
| | 10 | 100 | 100 | 100 | 100 |

### Toxizität der angemeldeten Zusammensetzung

**Tabelle 4**

| Dosis, mg/kg | Anzahl der Versuchstiere | | | Anzahl der gestorbenen Tiere, in % | | |
|---|---|---|---|---|---|---|
| | Angemeldete Zusammensetzung nach Anspruch 1 (ohne Platin) | Angemeldete Zusammensetzung nach Anspruch 2 (mit Zugabe des Platin-Komplexes) | Cis-Platin | Angemeldete Zusammensetzung nach Anspruch 1 (ohne Platin) | Angemeldete Zusammensetzung nach Anspruch 2 (mit Zugabe des Platin-Komplexes) | Cis- Platin |
| 15 | 5 | 5 | 5 | 0 | 0 | 4/80% |
| 30 | 5 | 5 | 5 | 0 | 0 | 5/100% |
| 60 | 5 | 5 | 5 | 0 | 0 | 5/100% |
| 90 | 5 | 5 | 5 | 2/40% | 0 | 5/100% |
| 120 | 5 | 5 | 5 | 5/100% | 1/20% | 5/100% |

### Virentötende Aktivität der angemeldeten Zusammensetzung (Ausführungsvariante 2) am Beispiel der Inhibierung der Virus-Hämoblastose

**Tabelle 5**

| Prozentanteil der gestorbenen Versuchstiere | | | |
|---|---|---|---|
| Angemeldete Zusammensetzung nach Beispiel 5 | Angemeldete Zusammensetzung nach Beispiel 8 | Angemeldete Zusammensetzung nach Beispiel 7 | Referenz (unbehandelte Tiere) |
| 20 | 8,5 | 17,1 | 100 |

### Antiblastomatöse Aktivität der angemeldeten platinhaltigen Zusammensetzung

**Tabelle 6**

| Art des Tumors | Angemeldete Zusammensetzung | Referenz (unbehandelte Tiere) |
|---|---|---|
| Mastozytom p-815 | 63,3 + 4,4 | 17,0+1,3 |

### Informationsquellen zum Stand der Technik

1. Patent RU Nr. 2181050, IPC 7 A61K33/18, A61K33/38. 2002. Antiblastomatöse und antibakterielle Zusammensetzung.
2. Patent RU Nr. 2074861, 1997.03.10 IPC 6 C07F15/00. Pharmazeutische Mittel auf Platin-Basis.
3. Patent RU Nr. 2114858, IPC 6 C07F15/00, A61 K31/295, 1998. Platin Cis-Diamindiimidazolyl Dichlorid-Derivate mit ausgeprägter antiproliferativer Aktivität und Verfahren zu ihrer Erzeugung.
4. Patent RU Nr. 2094048, IPC 6 A61 K33/38, veröff. am 27.10.97. Zytostatische und virentötende Zusammensetzung. Prototyp.

## Patentansprüche

1. Antiblastomatöse, antibakterielle und virentötende pharmazeutische Zusammensetzung mit Höllenstein, Hexamethylentetramin, Natriumthiosulfat und Wasser als Basis,
**dadurch gekennzeichnet,**
**dass** sie zusätzlich Alpha-Asparaginsäure oder Asparagin und Nikotinsäure mit folgendem Komponentenverhältnis in Gew. %, enthält:
| | |
|---|---|
| Höllenstein | 0,016-1,6, |
| Alpha-Asparaginsäure oder Asparagin | 0,07 - 0,70, |
| Nikotinsäure | 0,07 - 0,70, |
| Hexamethylentetramin | 0,03 - 1,4, |
| Natriumthiosulfat | 0,02 - 5,0 und |
| Wasser | Rest bis 100 |

2. Antiblastomatöse, antibakterielle und virentötende pharmazeutische Zusammensetzung mit Höllenstein, Hexamethylentetramin oder Imidazol und Wasser als Basis,
**dadurch gekennzeichnet,**
**dass** sie zusätzlich imidazolhaltige Platinverbindungen Cis-[Pt(NH₃)₂Im₂]Cl₂ oder [Pt(NH₂OH)₂Im₂]Cl₂ mit folgendem Komponentenverhältnis, in Gew. % enthält:
| | |
|---|---|
| Höllenstein | 0,016-1,6, |
| Hexamethylentetramin oder Imidazol | 0,03 - 1,4, |
| Cis-[Pt(NH₃)₂Im₂]Cl₂ oder Cis-[Pt(NH₂OH)₂Im₂]Cl₂ | 0,056 - 5,6, |
| Alpha-Asparaginsäure oder Asparagin | 0,07 - 0,70, |
| Nikotinsäure | 0,07 - 0,70 und |
| Wasser | Rest bis 100 |

## Claims

1. An antiblastomatous, antibacterial, virucidal pharmaceutical composition having silver nitrate, hexamethylene tetramine, sodium thiosulfate, and water as the basis,
**characterized in that** it additionally contains alpha-aspartic acid or aspartamic acid and nicotinic acid in the following component ratio in weight-%:
| | |
|---|---|
| silver nitrate | 0.016-1.6, |
| alpha-aspartic acid or aspartamic acid | 0.07-0.70, |
| hexamethylene tetramine | 0.07-0.70, |
| nicotinic acid | 0.03-1.4, |
| sodium thiosulfate | 0.02-5.0, and |
| water | remainder up to 100. |

2. An antiblastomatous, antibacterial, virucidal pharmaceutical composition having silver nitrate, hexamethylene tetramine or imidazole, and water as the basis,
**characterized in that** it additionally contains imidazole-containing platinum compounds Cis-[Pt(NH₃)₂Im₂]Cl₂ or [Pt(NH₂OH)₂Im₂]Cl₂ in the following component ratio in weight-%:
| | |
|---|---|
| silver nitrate | 0.016-1.6, |
| hexamethylene tetramine or imidazole | 0.03-1.4, |
| Cis-[Pt(NH₃)₂Im₂]Cl₂ or [Pt(NH₂OH)₂Im₂]Cl₂ | 0.056-5.6, |
| alpha-aspartic acid or aspartamic acid | 0.07-0.70, |
| nicotinic acid | 0.07-0.70, and |
| water | remainder up to 100. |

## Revendications

1. Composition pharmaceutique anti-blastomateuse, antibactérienne et antivirale comportant du nitrate d'argent, de l'hexaméthylènetétramine, du thiosulfate de sodium et de l'eau comme base, **caractérisée en ce qu'**elle contient en outre de l'acide alpha-aspartique ou de l'asparagine et de l'acide nicotinique selon le rapport suivant entre composants, en % en masse :
| | |
|---|---|
| Nitrate d'argent | 0,016-1,6 |
| Acide alpha-aspartique ou asparagine | 0,07-0,70 |
| Acide nicotinique | 0,07-0,70 |
| Hexaméthylènetétramine | 0,03 - 1,4 |
| Thiosulfate de sodium | 0,02 - 5,0 et |
| Eau | le reste jusqu'à 100. |

2. Composition pharmaceutique anti-blastomateuse, antibactérienne et antivirale comportant du nitrate d'argent, de l'hexaméthylènetétramine ou de l'imidazole et de l'eau en tant que base, **caractérisée en ce qu'**elle contient en outre des composés de platine contenant de l'imidazole Cis-[Pt(NH₃)₂Im₂]Cl₂ ou [Pt(NH₂OH)₂Im₂]Cl₂ selon le rapport suivant entre composants, en % en masse :
| | |
|---|---|
| Nitrate d'argent | 0,016-1,6 |
| Hexaméthylènetétramine ou imidazole | 0,03-1,4 |
| Cis-[Pt(NH₃)₂Im₂]Cl₂ ou Cis-[Pt(NH₂OH)₂Im₂]Cl₂ | 0,056 - 5,6 |
| Acide alpha-aspartique ou asparagine | 0,07 - 0,70 |
| Acide nicotinique | 0,07 - 0,70 et |
| Eau | le reste jusqu'à 100 |
